# EUROPÄISCHE PATENTANMELDUNG

(11) **EP 3 002 003 A1**
(43) Veröffentlichungstag der Anmeldung: **06.04.2016**
(21) Anmeldenummer: 14187075.8
(22) Anmeldetag: 30.09.2014
(51) Int. Cl.: A61K 8/33, C07C 47/28, C07C 47/38, C07C 45/27, C07C 45/29

(54) **Verwendung neuartiger cyclischer Carbaldehyde als Aromastoff**

(71) Anmelder: BASF SE, 67056 Ludwigshafen am Rhein (DE)
(72) Erfinder: Die Erfindernennung liegt noch nicht vor
(74) Vertreter: Reitstötter Kinzebach

(57) **Zusammenfassung**

Die vorliegende Erfindung betrifft neuartige cyclische Carbaldehyde, deren Herstellung und die Verwendung als Aromachemikalie, insbesondere als Riechstoff, sowie Aromastoffzusammensetzungen und Mittel, enthaltend diese Carbaldehyde.

## Beschreibung

Die vorliegende Erfindung betrifft neuartige cyclische Carbaldehyde, deren Herstellung und die Verwendung als Aromachemikalie, insbesondere als Riechstoff, sowie Aromastoffzusammensetzungen und Mittel, enthaltend diese Carbaldehyde.

### Hintergrund der Erfindung

In der Parfümindustrie besteht ein ständiger Bedarf an neuen Riechstoffen, die sich als Riechstoffkompositionen oder parfümierten Artikeln eignen. Die C₁₅-Aldehyde und dessen Mischungen stellen einen Beitrag zur Erweiterung des Repertoires in der Duftstoffindustrie, insbesondere der moschusartigen Riechstoffe, dar.

Insbesondere besteht ein Bedarf an moschusartigen Riechstoffen und Riechstoffkompositionen. Hierunter ist ein Geruch zu verstehen, der dem natürlich vorkommenden Moschusduft ähnlich ist.

### Zusammenfassung der Erfindung

Überraschenderweise wurde obige Aufgabe insbesondere durch Bereitstellung von Carbaldehyden der im Folgenden beschriebenen Formel X und vor allem durch Bereitstellung der speziellen Carbaldehyde der folgenden Formeln I, II und III gelöst, welche sich durch einen ausgeprägten Moschusduft auszeichnen:

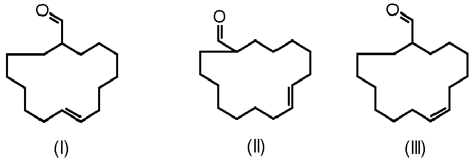

trans-Cyclopentadec-8-enylcarbaldehyd (I), *cis*-Cyclopentadec-7-enylcarbaldehyd (III), *trans-*Cyclopentadec-7-enylcarbaldehyd (II) oder Gemische davon, in Folge C₁₅-Aldehyde genannt, sind in der Literatur noch vollkommen unbekannt. Bei der Herstellung von Cyclohexadec-8-en-1-on durch Oxidation von Cyclohexadec-8,15-dien mit Lachgas, fallen C₁₅-Aldehyd (I) und C₁₅-Aldehyd **(III)** als Nebenprodukte in einem Verhältnis 40 : 60 an. C₁₅-Aldehyde **(I-III)** können darüber hinaus auch ausgehend von Cyclohexadec-8-en-1-on mittels einer mehrstufigen Synthese, die eine Wolff-Umlagerung enthält, hergestellt werden. Neben den bereits erwähnt *trans-* und *cis*-C₁₅-Aldehyden (I) und **(III)** wird hierbei noch der *trans*-C₁₅-Aldehyd **(II)** erhalten. Die Isomerenverteilung des durch Wolff-Umlagerung erhaltenen Produktgemisches liegt hier bei 43 **(I) :** 24 **(II) :** 33 **(III).** Es wurde herausgefunden, dass die C₁₅-Aldehyde **(I-III)** sehr gute olfaktorische Eigenschaften besitzen. Deren Geruchseigenschaften finden in der Literatur bisher keine Erwähnung.

### Figurenbeschreibung:

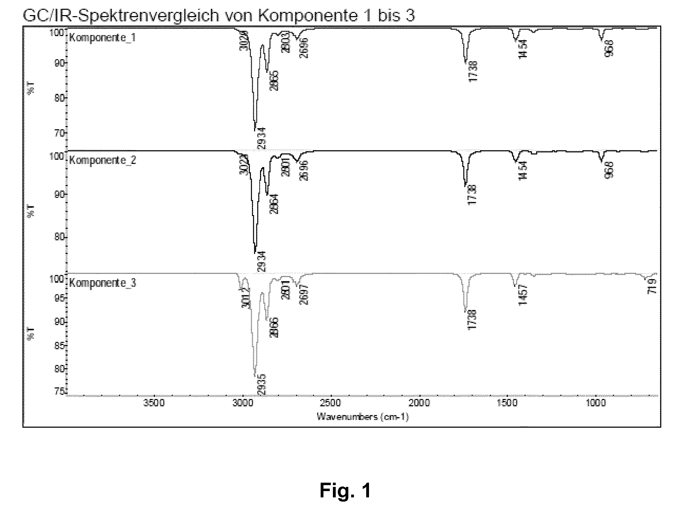

Figur 1 zeigt die GC/IR Spektren der erfindungsgemäßen Verbindungen I, II und II (dort bezeichnet als Komponenten 1, 2 und 3)

### Detaillierte Beschreibung der Erfindung

### a) Allgemeine Definitionen:

Eine "Aromachemikalie" ist ein Oberbegriff für Verbindungen die als "Riechstoff" und/oder als "Geschmacksstoff" einsetzbar sind.

Als "Riechstoff" sind im Sinne der vorliegenden Erfindung natürliche oder synthetische Stoffe mit Eigengeruch zu verstehen.

Als "Geschmacksstoff" sind im Sinne der vorliegenden Erfindung natürliche oder synthetische Stoffe mit Eigengeschmack zu verstehen.

Der "Geruch" oder die "olfaktorische Wahrnehmung" ist im Sinne der vorliegenden Erfindung die Interpretation der Sinneserregungen, die von den Chemorezeptoren der Nase oder anderen Geruchsorganen an das Gehirn eines Lebewesens geliefert werden. Der Geruch kann folglich eine Sinneswahrnehmung der Nase von Riechstoffen sein, die beim Einatmen erfolgt. Die Luft dient hierbei als Geruchsträger.

Als "Duft" ist im Sinne der vorliegenden Erfindung ein wohlriechender Geruch zu verstehen. Entsprechendes gilt für einen erfindungsgemäßen "Duftstoff".

Ein "Parfüm" ist im Sinne der vorliegenden Erfindung ein Gemisch aus Riechstoffen und Träger, wie insbesondere einem Alkohol.

Eine "Parfümzusammensetzung" ist im Sinne der vorliegenden Erfindung ein Parfüm, welches unterschiedliche Mengen harmonisch aufeinander abgestimmter Einzelkomponenten enthält. Die Eigenschaften der Einzelbestandteile werden genutzt, um in der Kombination ein neues Gesamtbild zu schaffen, wobei die Charakteristika der Ingredienzen in den Hintergrund treten, ohne jedoch unterdrückt zu werden.

Ein "Parfümöl" ist im Sinne der vorliegenden Erfindung ein konzentriertes Gemisch aus mehreren Riechstoffen, die z.B. in alkoholischen Lösungen zur Parfümierung verschiedener Produkte gebraucht werden.

Ein "Duftthema" ist im Sinne der vorliegenden Erfindung die vorherrschende Duftnote in einer Riechstoffzusammensetzung.

Die "Kopfnote" ist im Sinne der vorliegenden Erfindung die erste Phase des Duftablaufs eines Parfüms. Sie spielt die ausschlaggebende Rolle beim ersten Eindruck, beim Öffnen des Flakons und beim Auftragen des Parfüms auf die Haut. Die Aufgabe der Kopfnote ist es, Interesse für das Parfüm allgemein zu wecken und für Aufmerksamkeit zu sorgen. Deshalb ist ein außergewöhnlicher Charakter häufig wichtiger als eine ausgefeilte Harmonie. Die Kopfnote wird naturgemäß von leichtflüchtigen Riechstoffen bestimmt.

"Modifizieren" bedeutet im Sinne der vorliegenden Erfindung, das Grundthema einer Riechstoffzusammensetzung mit zusätzlichen oder anderen Akkorden und Geruchsnuancen zu versehen.

"Akkorde" entstehen im Sinne der vorliegenden Erfindung durch das Zusammenfügen verschiedener Riechstoffe, die sich somit zu neuen Geruchsbildern vereinigen. Die Anzahl der eingesetzten Riechstoffe kann von zwei bis zu hundert Verschiedene reichen.

Eine "organoleptisch/sensorisch wirksame Menge" im Sinne der vorliegenden Erfindung ist die Menge eines Riechstoffes, die ausreicht, um anregend auf ein Sinnesorgan beziehungsweise anregend auf einen sensorischen Rezeptor zu wirken.

### b) Spezielle Ausführungsformen der Erfindung

Vorliegende Erfindung betrifft insbesondere folgende Gegenstände:
1. Carbaldehyde der allgemeinen Formel X 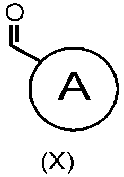 worin A für einen cycloaliphatischen Kohlenwasserstoffrest mit m Ringkohlenstoffatomen, wobei m für einen ganzzahligen Wert von 13 bis 17, wie z.B. 13, 14, 15, 16 oder 17, insbesondere 15, steht, und gegebenenfalls n C=C-Doppelbindungen aufweist, wobei n für einen ganzzahligen Wert von 1, 2 oder 3 steht, insbesondere 1, in stereoisomerenreiner Form oder in Form von Stereioisomerengemischen, enthaltend wenigstens zwei stereoisomere Formen einer solchen Carbaldehyds; sowie Stoffgemische enthaltend wenigstens 2 wie z.B. 2, 3, 4 oder 5, insbesondere 2 oder 3, derartige Carbaldehyde in jeweils stereoisomerenreiner Form oder als Stereoisomerengemisch. Zusätzlich sind Verbindungen bevorzugt, worin im Falle von n=1 die Carbaldehydgruppe und die Ring-C=C-Doppelbindung 4 bis 7 Ring-C-Atome voneinander entfernt sind. Stoffgemische können, wenn n nicht für 0 steht auch Konstitutionsisomere umfassen. Falls n für 2 oder 3 steht dann sind die Doppelbindungen nicht kumuliert.
2. Verbindungen nach Ausführungsform 1, worin n für 1 steht und/oder m für 15 steht, Verbindungen mit n =1 und m = 15 sind besonders bevorzugt.
3. Verbindung nach Ausführungsform 2, ausgewählt unter den isomeren Verbindungen der Formeln I, II und III 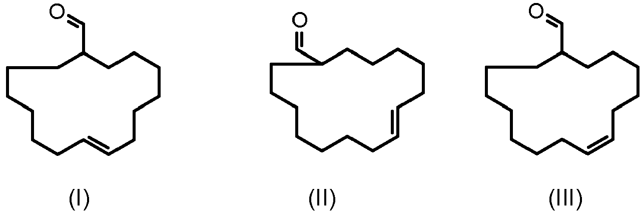 und den stereoisomeren Formen davon.
4. Stoffgemisch umfassend wenigsten eine Verbindung nach einer der Ausführungsformen 1 bis 3
5. Verwendung wenigstens eines Stoffes oder Stoffgemisches nach einer der vorhergehenden Ausführungsformen als Aromachemikalie, insbesondere als Riechstoff.
6. Verwendung nach Ausführungsform 5 in Mitteln, ausgewählt unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.
7. Verwendung nach einer der Ausführungsformen 5 und 6 eines Gemisches, im Wesentlichen enthaltend trans-Cyclopentadec-8-enylcarbaldehyd (I).
8. Verwendung nach Ausführungsform 7, eines Gemisches enthaltend *trans*-Cyclopentadec-8-enylcarbaldehyd (I), trans-Cyclopentadec-7-enylcarbaldehyd (II) und *cis-*Cyclopentadec-7-enylcarbaldehyd (III), wobei im Gemisch der Anteil von trans-Cyclopentadec-8-enylcarbaldehyd (I) bezogen der Summe der Komponenten der Formeln I, II und III mindestens 65% wie z.B. 65-100%, 75-98% oder 85-95% beträgt.
9. Verwendung nach Ausführungsform 5 oder 6 eines Gemisches enthaltend die Verbindungen der Formeln I, II und III, wobei das Gewichtsverhältnis von I : II : III im Bereich von 0,3-0,5 : 0,2-0,3 : 0,3-0,4 liegt.
10. Verwendung nach Ausführungsform 9 eines Gemisches enthaltend die Verbindungen der Formeln I, II und III in einem Gewichtsverhältnis von I : II : III, von etwa 43 : 24 : 33.
11. Verwendung nach einer der Ausführungsformen 5 bis 10 zur Erzeugung einer Moschusnote in einer Riechstoffzusammensetzung.
12. Verwendung nach Ausführungsform 11, zum Vermitteln, Modifizieren und/oder Verstärken einer Moschusduftnote in einer Riechstoffzusammensetzung durch Beimischen einer sensorisch wirksamen Menge wenigstens eines Stoffs oder ein Stoffgemisches gemäß der Definition in einer der Ausführungsformen 1 bis 10.
13. Verfahren zur Herstellung von eine Verbindung der allgemeinen Formel X 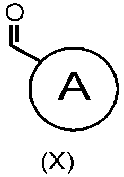 worin A für einen cycloaliphatischen Kohlenwasserstoffrest mit m Ringkohlenstoffatomen, wobei m für einen ganzzahligen Wert von 13 bis 17, wie z.B. 13, 14, 15, 16 oder 17, insbesondere 15, steht, und gegebenenfalls n C=C-Doppelbindungen aufweist, wobei n für einen ganzzahligen Wert von 1, 2 oder 3 steht, wobei man
   a) eine cycloaliphatischen Verbindung der Formel XI 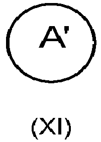 worin A' für einen cycloaliphatischen Kohlenwasserstoffrest mit m+1 Ringkohlenstoffatomen, wobei m für einen ganzzahligen Wert von 13 bis 17 steht, und gegebenenfalls n+1 C=C-Doppelbindungen aufweist, wobei n für einen ganzzahligen Wert von 1, 2 oder 3, insbesondere 1, steht, der mit Distickstoffmonoxid (N₂O) oxidiert wird; und
   b) wenigstens eine Verbindung der Formel X aus dem Reaktionsgemisch isoliert.
14. Verfahren nach Ausführungsform 13, wobei man die Verbindung der Formel X durch Destillation und gegebenenfalls anschließende Chromatographie isoliert.
15. Verfahren nach Ausführungsform 13 oder 14, wobei man als Edukt der Formel XI ein 1,9-Cyclohexadecadien einsetzt und trans-Cyclopentadec-8-enylcarbaldehyd (I) und/oder *cis-*Cyclopentadec-7-enylcarbaldehyd (III), isoliert.
16. Verfahren nach einer der Ausführungsformen 13 bis 15, wobei man die Verbindung der Formel (I) und der Formel (III) mittels fraktionierter Destillation aus einem Reaktionsgemisch isoliert, worin das Gewichtsverhältnis von (I) plus (III) zu Cyclohexadeca-1,8-dien mindestens 0.01, wie z.B. 0.01-0.1, 0.03-0.095 oder 0.06-0.09 beträgt.
17. Verfahren nach einer der Ausführungsformen 13 bis 15, wobei man trans-Cyclopentadec-8-enylcarbaldehyd (I) mittels chromatischer Aufreinigung aus einem Gemisch isoliert, welches durch fraktionierte Destillation erhalten wurde, und einen Gehalt an (I) plus (III) von mindestens 5 Gew.-%, wie z.B. 5-50%, 20-48 oder 30-45%, aufweist.
18. Verfahren zur Herstellung von eine Verbindung der allgemeinen Formel X 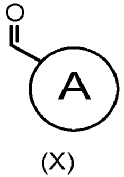 worin A für einen cycloaliphatischen, Kohlenwasserstoffrest mit m Ringkohlenstoffatomen, wobei m für einen ganzzahligen Wert von 13 bis 17 steht, und gegebenenfalls n C=C-Doppelbindungen aufweist, wobei n für einen ganzzahligen Wert von 1, 2 oder 3 steht, wobei man
   a) eine cycloaliphatischen Verbindung der Formel XII 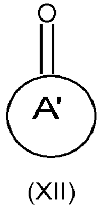 worin A' für einen cycloaliphatischen, Kohlenwasserstoffrest mit m+1 Ringkohlenstoffatomen, wobei m für einen ganzzahligen Wert von 13 bis 17, wie z.B. 13, 14, 15, 16 oder 17, insbesondere 15, steht, und gegebenenfalls n+1 C=C-Doppelbindungen aufweist, wobei n für einen ganzzahligen Wert von 1, 2 oder 3, insbesondere 1, steht, mit NaH und Methylformiat zum korrespondierenden cyclischen Formylketon XIII 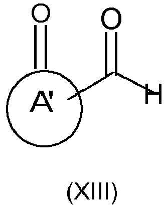 umsetzt; worin A' wie oben definiert ist, wobei Keto- und Formylgruppe bevorzugt an benachbarten Ring-C-Atomen gebunden sind;
   b) das gebildete Formylketon mit Triethylamin und 4-Acetamidobenzoesäureazid zum korrespondierende Diazoketon XIV umsetzt; 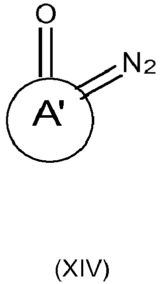 worin A' wie oben definiert ist, wobei Keto- und Diazogruppe bevorzugt an benachbarten Ring-C-Atomen gebunden sind;
   c) aus dem gebildete Diazoketon unter den Bedingungen einer Wolff-Umlagerung N₂ abspaltet und in Gegenwart eines Alkoholszum korrespondierenden Ester der Formel XV 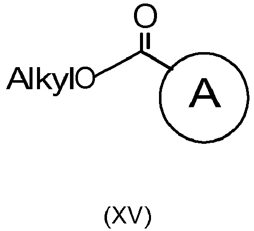 umsetzt; worin A wie oben definiert ist
   d) den so gebildeten Ester zum korrespondierenden Alkohol XVI 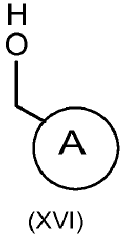 reduziert; worin A wie oben definiert ist;
   e) den so gebildeten Alkohol zum Carbaldehyd der Formel X oxidiert;
   f) und gegebenenfalls das Carbaldehyd der Formel X aus dem Reaktionsgemisch isoliert.
19. Verfahren nach Ausführungsform 18, wobei man (E/Z)-Cyclohexadec-8-enon als Verbindung der Formel XI in Stufe a) einsetzt und *trans*-Cyclopentadec-8-enylcarbaldehyd (I), *trans-* Cyclopentadec-7-enylcarbaldehyd (II) und *cis*-Cyclopentadec-7-enylcarbaldehyd (III), in Stufe e) erhält.
20. Verfahren nach Ausführungsform 15, wobei man Verbindungen der Formel (I), (II) oder (III) oder Gemische davon in Stufe f) durch chromatische Aufreinigung eines Reaktionsgemischs aus Stufe e) erhält, wobei dessen Gehalt an (I), (II) und (III) in Summe vorzugsweise mindestens 50 Gew.-% wie z.B. 50-100 Gew.-%, 60-99 Gew.-% oder 70-95 Gew.-% beträgt.
21. Riechstoffzusammensetzung, enthaltend wenigstens einen Stoff oder ein Stoffgemisch gemäß der Definition in einer der Ausführungsformen 1 bis 10 oder hergestellt nach einem der Verfahren gemäß den Ausführungsformen 13 bis 20.
22. Zusammensetzung nach Ausführungsform 21, enthaltend den Stoff oder das Stoffgemisch in einem Gewichtsanteil von 0,01 bis 99,9 Gew.-% bezogen auf das Gesamtgewicht der Zusammensetzung.
23. Mittel enthaltend wenigstens einen Stoff oder ein Stoffgemisch gemäß der Definition in einer der Ausführungsformen 1 bis 10 oder hergestellt nach einem der Verfahren gemäß den Ausführungsformen 13 bis 20.
24. Mittel nach Ausführungsform 23, enthaltend den Stoff oder das Stoffgemisch in einem Gewichtsanteil von 0,01 bis 99,9 Gew.-%, 1 bis 80 Gew.-%, 2 bis 50 Gew.-%, 3 bis 25 oder 5 bis 15 Gew.-% bezogen auf das Gesamtgewicht der Zusammensetzung.
25. Mittel nach Ausführungsform 23 oder 24, ausgewählt unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.

### c) Weiter Ausgestaltungen der Erfindung

### c1) Riechstoffzusammensetzungen:

Einem weiteren Aspekt zufolge werden die erfindungsgemäß verwendeten Riechstoffe insbesondere zwecks effizienterer Handhabung und Dosierung, auch als Riechstoffmischungen mit Verdünnungs- oder Lösungsmitteln eingesetzt. Hierbei wird der Anteil der Riechstoffe, bezogen auf die Summe von Riechstoffen und Lösungsmittel, in Gew.-% angegeben.

### Lösungsmittel:

Ein "Lösungsmittel" dient im Sinne der vorliegenden Erfindung der Verdünnung der erfindungsgemäß zu verwendenden Riechstoffe oder der erfindungsgemäßen Riechstoffzusammensetzung, ohne eigene riechende Eigenschaften zu besitzen. Manche Lösungsmittel haben zugleich fixierende Eigenschaften.

Die erfindungsgemäße Verbindung der Formel (X) oder ein oben definiertes Stoffgemisch aus mehreren Verbindungen/Isomeren der Formel (X) kann zu 0,1 bis 99 Gew-% einem Verdünnungs- oder Lösungsmittel beigemischt werden. Bevorzugt sind mindestens 40 Gew.- %ige Lösungen, weiter bevorzugt mindestens 50 Gew.-%ige Lösungen, weiterhin bevorzugt mindestens 60 Gew.-%ige Lösungen, weiter bevorzugt mindestens 70 Gew.-%ige Lösungen, insbesondere bevorzugt mindestens 80 Gew.-%ige Lösungen, weiterhin insbesondere bevorzugt mindestens 90 Gew.-%ige Lösungen, vorzugsweise in olfaktorisch akzeptablen Lösungsmitteln.

Bevorzugte olfaktorisch akzeptable Lösungsmittel sind Ethanol, Dipropylenglycol (DPG), Propylenglycol, 1,2-Butylenglycol, Glycerin, Diethylenglycolmonoethylether, Diethylphthalat (DEP), Isopropylmyristat (IPM), Triethylcitrat (TEC), Benzylbenzoat (BB) und Benzylacetat. Hierbei wiederum bevorzugt sind Ethanol, Diethylphthalat, Propylenglycol, Dipropylenglycol, Triethylcitrat, Benzylbenzoat und Isopropylmyristat.

Eine "Riechstoffzusammensetzung" ist im Sinne der vorliegenden Erfindung eine Mischung, die neben einer erfindungsgemäßen Verbindung der Formel (X) oder einem oben definierten Stoffgemisch aus mehreren Verbindungen/Isomeren der Formel (X) mindestens einen weiteren Riechstoff umfasst. Insbesondere kann es sich bei einer solchen Riechstoffkomposition um eine Parfümkomposition (ein Parfümöl) handeln.

Erfindungsgemäße Riechstoffkompositionen enthalten, bezogen auf die Gesamtmenge der Riechstoffkomposition, z.B. eine Menge von einer erfindungsgemäßen Verbindung der Formel (X) oder eines oben definierten Stoffgemischs aus mehreren Verbindungen/Isomeren der Formel (X) von 0,01 bis 65 Gew.-%, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%. Das Gewichtsverhältnis von erfindungsgemäßer Verbindung/- erfindungsgemäßen Verbindungen der Formel X zu der Gesamtmenge an weiteren Riechstoffen liegt z.B. im Bereich von 1:1000 bis 1:0,5 liegt, vorzugsweise im Bereich von 1:700 bis 1:1, besonders bevorzugt im Bereich von 1:500 bis 1:10.

Erfindungsgemäße Riechstoffkompositionen enthalten, bezogen auf die Gesamtmenge der Riechstoffkomposition, z.B. eine Menge von erfindungsgemäßer Verbindung/ erfindungsgemäßen Verbindungen der Formel X von 0,01 bis 65 Gew.-% und, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%. Das Gewichtsverhältnis von erfindungsgemäßer Verbindung/erfindungsgemäßen Verbindungen der Formel X zu der Gesamtmenge an weiteren (davon verschiedenen) Riechstoffen liegt z.B. im Bereich von 1:1000 bis 1:0,5 liegt, vorzugsweise im Bereich von 1:700 bis 1:1, besonders bevorzugt im Bereich von 1:500 bis 1:10.

### Weitere Riechstoffe:

Erfindungsgemäße Riechstoffkompositionen enthalten neben der erfindungsgemäßen Verbindung/ den erfindungsgemäßen Verbindungen der Formel X zumindest einen weiteren Riechstoff, vorzugsweise 2, 3, 4, 5, 6, 7, 8 oder mehr weitere Riechstoffe, wobei weitere Riechstoffe z.B. ausgewählt sind unter:
Alpha-Hexylzimtaldehyd, 2-Phenoxyethylisobutyrat (Phenirat¹), Dihydromyrcenol (2,6-Dimethyl-7-octen-2-ol), Methyldihydrojasmonat (vorzugsweise mit einem Gehalt an cis-Isomerem von mehr als 60 Gew.-%) (Hedione⁹, Hedione HC⁹), 4,6,6,7,8,8-Hexamethyl-1,3,4,6,7,8-hexahydrocyclopenta[g]benzopyran (Galaxolid³), Tetrahydrolinalool (3,7-Dimethyloctan-3-ol), Ethyllinalool, Benzylsalicylat, 2-Methyl-3-(4-tert-butylphenyl)propanal (Lilial²), Zimtalkohol, 4,7-Methano-3a,4,5,6,7,7a-hexahydro-5-indenylacetat und/oder 4,7-Methano-3a,4,5,6,7,7a-hexahydro-6-indenylacetat (Herbaflorat¹), Citronellol, Citronellylacetat, Tetrahydrogeraniol, Vanillin, Linalylacetat, Styrolylacetat (1-Phenylethylacetat), Octahydro-2,3,8,8-tetramethyl-2-acetonaphthon und/oder 2-Acetyl-1,2,3,4,6,7,8-octahydro-2,3,8,8-tetramethyl-naphtalin (Iso E Super³), Hexylsalicylat, 4-tert-Butylcyclohexylacetat (Oryclone¹), 2-tert-Butylcyclohexylacetat (Agrumex HC¹), Alphalonon (4-(2,2,6-Trimethyl-2-cyclohexen-1-yl)-3-buten-2-on), n-alpha-Methylionon, alpha-Isomethylionon, Coumarin, Terpinylacetat, 2-Phenylethylalkohol, 4-(4-Hydroxy-4-methylpentyl)-3-cyclohexencarboxaldehyd (Lyral³), alpha-Amylzimtaldehyd, Ethylenbrassylat, (E)- und/oder (Z)-3-Methylcyclopentadec-5-enon (Muscenon⁹), 15-Pentadec-11-enolid und/oder 15-Pentadec-12- enolid (Globalide¹), 15-Cyclopentadecanolid (Macrolide¹), 1-(5,6,7,8-Tetrahydro-3,5,5,6,8,8-hexamethyl-2-naphthalenyl) ethanon (Tonalid¹⁰), 2-Isobutyl-4-methyltetrahydro-2H-pyran-4-ol (Florol⁹), 2-Ethyl-4- (2,2,3-trimethyl-3-cyclopenten-1-yl)-2-buten-1-ol (Sandolen¹), cis-3-Hexenylacetat, trans-3-Hexenylacetat, trans-2,cis-6-Nonadienol, 2,4-Dimethyl- 3-cyclohexencarboxaldehyd (Vertocitral¹), 2,4,4,7-Tetramethyl-oct-6-en-3-on (Claritone¹), 2,6-Dimethyl-5-hepten-1-al (Melonal²), Borneol, 3-(3-Isopropylphenyl)butanal (Florhydral²), 2-Methyl-3-(3,4-methylendioxyphenyl)propanal (Helional³), 3-(4-Ethylphenyl)-2,2-dimethylpropanal (Florazon¹), 7-Methyl-2H-1,5-benzodioxepin-3(4H)-on (Calone¹⁹⁵¹⁵), 3,3,5-Trimethylcyclohexylacetat (vorzugsweise mit einem Gehalt an cis-Isomeren von 70 Gew.-%) oder mehr und 2,5,5-Trimethyl-1,2,3,4,4a,5,6,7-octahydronaphthalin-2-ol (Ambrinol S¹). Die vorangehend genannten Riechstoffe werden im Rahmen der vorliegenden Erfindung demnach bevorzugt mit erfindungsgemäßen Mischungen kombiniert.

Sofern vorstehend Handelsnamen angegeben sind, beziehen sich diese auf folgende Quellen:
1 Handelsname Symrise GmbH, Deutschland;
2 Handelsname Givaudan AG, Schweiz;
3 Handelsname International Flavors & Fragrances Inc., USA;
5 Handelsname Danisco Seillans S.A., Frankreich;
9 Handelsname Firmenich S.A., Schweiz;
10 Handelsname PFW Aroma Chemicals B.V., Niederlande.

Weitere Riechstoffe, mit denen Hexadeca-8,15-dienal z.B. zu einer Riechstoffkomposition kombiniert werden können, finden sich z.B. in S. Arctander, Perfume and Flavor Chemicals, Vol. I und II, Montclair, N. J., 1969, Selbstverlag oder K. Bauer, D. Garbe und H. Surburg, Common Fragrance and Flavor Materials, 4rd. Ed., Wiley- VCH, Weinheim 2001. Im Einzelnen seien genannt:
Extrakte aus natürlichen Rohstoffen wie etherischen Ölen, Concretes, Absolues, Resine, Resinoide, Balsame, Tinkturen wie z.B.
Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptuscitriodora Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyl-oxyacetaldehyd; 1-(1-Methoxy-propoxy)-(E/Z)-3-hexen; der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat;
3-Octylacetat; 1-Octen-3-ylacetat; Ethylbutyrat; Butylbutyrat; Isoamylbutyrat; Hexylbutyrat; (E)-und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z.B. Geraniol; Nerol; Linalool; Lavadulol; Nerolidol; Farnesol; Tetrahydrolinalool; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z.B. Geranial; Neral; Citronellal; 7-Hydroxy-3,7-dimethyloctanal; 7- Methoxy-3,7-dimethyloctanal; 2,6,1 0-Trimethyl-9-undecenal; Geranylaceton; sowie die Dimethyl- und Diethylacetale von Geranial, Neral, 7-Hydroxy-3,7-dimethyloctanal; der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl- 2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1-cyclohexen-1-yl)-2-butenal; Nootkaton; Dihydronootkaton; 4,6,8-Megastigmatrien-3-on; alpha-Sinensal ; beta-Sinensal ; acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3 trimethyl-3-cyclopent-1-yl)-4-penten-2-ol; 1-(2,2,6-Trimethylcyclohexyl)pentan-3-ol; 1-(2,2,6Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclo-dodecan; alpha-Cedrenepoxid; 3a,6,6,9a Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclo-pentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4 cyclopenta-decenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 7-Cyclohexadecen-1-on; (7/8)-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z.B. 1-(3,3-Dimethylcyclohexyl)-4-penten-1-on; 2,2-Dimethyl-1-(2,4-di methyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclo- pentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7 Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z.B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol, 2-Phenylethylalkohol, 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropa-aldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4 Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; 2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4 indanylmethylketon; 1-[2,3-dihydro-1,1,2,6-tetramethyl-3-(1-methylethyl)-1 H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methyl-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; 2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2Hfuran-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

### c2) Riechstoffhaltige Artikel

Erfindungsgemäßes Hexadeca-8,15-dienal oder erfindungsgemäße Riechstoffkompositionen können in eine Reihe von Produkten eingearbeitet bzw. auf solche Produkte appliziert werden.

Erfindungsgemäße Riechstoffe können bei der Herstellung parfümierter Artikel eingesetzt. Die olfaktorischen Eigenschaften ebenso wie die stofflichen Eigenschaften (wie Löslichkeit in gängigen Lösungsmitteln und Kompatibilität mit gängigen weiteren Bestandteilen derartiger Produkte) sowie die toxikologische Unbedenklichkeit der erfindungsgemäßen Riechstoffe unterstreichen ihre besondere Eignung für die genannten Einsatzzwecke. Die positiven Eigenschaften tragen dazu bei, dass die erfindungsgemäß verwendeten Riechstoffe und die erfindungsgemäßen Riechstoffkompositionen besonders bevorzugt in Parfümerzeugnissen, Körperpflegeprodukten, Hygieneartikeln, Textilwaschmittel sowie in Reinigungsmitteln für feste Oberflächen eingesetzt werden.

Der parfümierte Artikel ist z.B. ausgewählt unter Parfümerzeugnissen, Körperpflegeprodukten, Hygieneartikeln, Textilwaschmitteln und Reinigungsmitteln für feste Oberflächen. Bevorzugte erfindungsgemäße parfümierte Artikel sind weiterhin ausgewählt unter:
Parfümerzeugnissen, ausgewählt unter Parfüm-Extrakten, Eau de Parfums, Eau de Toilettes, Eau de Colognes, Eau de Solide, Extrait Parfum, Luftverbesserern in flüssiger, gelartiger oder auf einem festen Träger aufgebrachter Form, Aerosolsprays, Duftreinigern und -ölen;
Körperpflegeprodukten, ausgewählt unter Rasierwässern, Pre-shave-Produkten, Splash-Colognes, festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und - lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigende Haarlotionen, Haarspülungen, Haarshampoo, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara, Zahnpasta, Zahnseide;
Hygieneartikeln, ausgewählt unter Kerzen, Lampenölen, Räucherstäbchen, Insektiziden, Repellentien, Treibstoffen, Rostentfernern, parfümierten Erfrischungstüchern, Achselpads, Babywindeln, Damenbinden, Toilettenpapier, Kosmetiktüchern, Taschentüchern, Spülmaschinendeo;
Reinigungsmitteln für feste Oberflächen, ausgewählt unter parfümierten sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Wachsen und Polituren wie Möbelpolituren, Fußbodenwachsen, Schuhcremes, Desinfektionsmitteln, Oberflächendesinfektionsmitteln und Sanitärreinigern, Bremsenreinigern, Rohrreinigern, Entkalkern, Grill- und Backofenreinigern, Algen- und Moosentfernern, Schimmelentfernern, Fassadenreinigungsmitteln;
Textilwaschmitteln, ausgewählt unter flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten.

Einem weiteren Aspekt zufolge sind die erfindungsgemäß verwendeten Riechstoffe und die erfindungsgemäßen Riechstoffkompositionen für den Einsatz in tensidhaltigen parfümierten Artikeln geeignet. Gesucht werden nämlich - insbesondere für die Parfümierung von tensidhaltigen Formulierungen wie zum Beispiel Reinigungsmitteln (insbesondere Geschirrspülmittel und Allzweckreiniger) - häufig Riechstoffe und/oder Riechstoffkompositionen mit einer Rosenkopfnote und ausgeprägter Natürlichkeit.

Einem weiteren Aspekt zufolge können erfindungsgemäß verwendete Riechstoffe und erfindungsgemäße Riechstoffkompositionen als Mittel zum Versehen von (a) Haaren oder (b) textilen Fasern mit der Geruchsnote rosig verwendet werden.

Die erfindungsgemäß zu verwendenden Riechstoffe und erfindungsgemäße Riechstoffkompositionen eignen sich daher besonders gut für den Einsatz in tensidhaltigen parfümierten Artikeln.

Bevorzugt ist es, wenn der parfümierte Artikel einer der folgenden ist:
- ein saures, alkalisches oder neutrales Reinigungsmittel, das insbesondere aus der Gruppe ausgewählt ist bestehend aus Allzweckreinigern, Fußbodenreinigern, Fensterglasreinigern, Geschirrspülmitteln, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Flecken-entfernern, Wäscheweichspülern, Waschseifen, Waschtabletten, Desinfektionsmitteln, Oberflächendesinfektionsmitteln,
- ein Luftverbesserer in flüssiger, gelartiger oder auf einem festen Träger aufgebrachter Form oder als Aerosolspray,
- ein Wachs oder eine Politur, die insbesondere aus der Gruppe ausgewählt ist bestehend aus Möbelpolituren, Fußbodenwachsen und Schuhcremes, oder
- ein Körperpflegemittel, das insbesondere aus der Gruppe ausgewählt ist bestehend aus Duschgelen und Shampoos Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und - lotionen, After-sun-cremes und -lotionen, Handcremes und -lotionen, Fußcremes und -lotionen, Enthaarungscremes und -lotionen, After-shave-Cremes und -lotionen, Bräunungscremes und - lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik.

Inhaltstoffe, mit denen erfindungsgemäß verwendete Riechstoffe oder erfindungsgemäße Riechstoffkompositionen vorzugsweise kombiniert werden können, sind beispielsweise: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, a-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Einem weiteren Aspekt zufolge verwendet man die Riechstoffe bei der Herstellung der parfümierten Artikel in flüssiger Form, unverdünnt oder mit einem Lösungsmittel verdünnt oder in Form einer Riechstoffkomposition. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw. Für die genannten Lösungsmittel gilt, dass diese, im Falle des Vorhandenseins eigener olfaktorischer Eigenschaften, ausschließlich dem Bestandteil "Lösungsmittel" und nicht den "Riechstoffen" zuzuordnen sind.

Die in den erfindungsgemäßen parfümierten Artikeln enthaltenen Riechstoffe und/oder Riechstoffkompositionen können dabei in einer Ausführungsform an einem Trägerstoff absorbiert sein, der sowohl für eine feine Verteilung des Riechstoffs oder der Riechstoffkomposition im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die erfindungsgemäß verwendeten Riechstoffe und die erfindungsgemäßen Riechstoffkompositionen können auch mikroverkapselt, sprühgetrocknet, als Einschluss-Komplexe oder als Extrusions-Produkte vorliegen und in dieser Form dem zu parfümierenden Produkt, bzw. Artikel, hinzugefügt werden. Die Eigenschaften können durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung kann beispielsweise durch das sogenannte Koazervationsverfahren mit Hilfe von Kapselmaterialien, z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von Riechstoffkompositionen und Cyclodextrinen oder Harnstoffderivaten in ein geeignetes Lösungsmittel, z.B. Wasser, hergestellt werden. Extrusionsprodukte können durch Verschmelzen erfindungsgemäß verwendeter Riechstoffe und erfindungsgemäßer Riechstoffkompositionen mit einem geeigneten wachsartigen Stoff und durch Extrusion mit nachfolgender Erstarrung, ggf. in einem geeigneten Lösungsmittel, z.B. Isopropanol, hergestellt werden.

### c3) Herstellung erfindungsgemäßer Riechstoffe

Die erfindungsgemäßen Carbaldehyde der Formel X 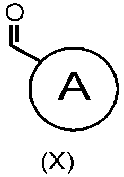 worin A wie oben definiert ist und für einen cyclischen C₁₃ bis C₁₇ Rest, insbesondere C₁₃-, C₁₅-, oder C₁₇-Rest steht; sind ausgehend von an sich bekannten cyclischen Ketonen oder cyclischen Olefin-Ausgangsstoffen erhältlich.

Folgende Synthesewege seien exemplarisch zur weiteren Erläuterung der Erfindung näher beschrieben:

### Syntheseweg 1: Carbaldehyd-Synthese durch N₂O-Oxidation

Ausgehend vom korrespondierenden von aliphatischen Carbocyclus, insbesondere einen cyclischen einfach oder mehrfach ungesättigen Olefin XI (d.h. im Vergleich zum cyclischen Rest A im Produkt der Formel X enthält die Ausgangsverbindung XI eine zusätzliche C=C-Doppelbindung und ein zusätzliches Ring-Kohlenstoffatom) sind Verbindungen der Formel X durch Distickstoffmonoxid Oxidation zugänglich, wie z.B. beschrieben in der WO2010/086313.

Beispiel für eine geeignete Ausgangsverbindung (die sowohl als in stereoisomerenreiner Form als auch in Form von Stereoisomeren-Gemischen eingesetzt werden kann) zur Herstellung von Verbindungen der Formel (X), worin A für einen einfach ungesättigten C₁₅-Rest steht, ist Cyclohexadeca-1,9-dien das entweder käuflich erhältlich ist oder gemäß Beispiel 2 der WO 2012/084673 hergestellt werden kann.

Insbesondere wird dabei ein cyclisches Olefin durch Umsetzung mit Distickstoffmonoxid oxidiert. Distickstoffmonoxid kann dabei rein oder ggf. im Gemisch mit anderen bei Reaktionsbedingungen gasförmigen Substanzen, wie z.B. Kohlenstoffdioxid zur Verdünnung eingesetzt werden.

Dabei kann für die Umsetzung des cyclischen Olefins mit Distickstoffmonoxid in Substanz oder in Gegenwart mindestens eines geeigneten Lösungsmittels oder Verdünnungsmittels erfolgen. Vorzugsweise erfolgt die Umsetzung in Substanz. Im Wesentlichen alle gängigen Lösungs- und/oder Verdünnungsmittel sind hierbei geeignet, jedoch unter der Maßgabe, dass sie weder eine C-C-Doppelbindung, noch eine C-C-Dreifachbindung, noch eine Aldehydgruppe aufweisen. Als geeignete Lösungsmittel sind unter anderem zu nennen: cyclische Alkane, beispielsweise Cyclohexan, Cyclopentan, Cyclooctan, Cyclododecan oder gesättigte aliphatische oder aromatische, gegebenenfalls alkylsubstituierte Kohlenwasserstoffe zu nennen,

Die Temperatur bei der Umsetzung liegt z.B. bei 140 bis 350°C, wie insbesondere bei 180 bis 320 °C oder bei 200 bis 300 °C. Es ist auch möglich, die Umsetzung bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden. Insbesondere ist die Umsetzungstemperatur aber im Wesentlichen konstant.

Der Druck bei der Umsetzung des cyclischen Olefins mit Distickstoffmonoxid liegt insbesondere höher als der Eigendruck des Edukt bzw. Produktgemisches bei der gewählten Reaktionstemperatur oder den gewählten Reaktionstemperaturen. Der Druck liegt z.B. bei 1 bis 1000 bar, wie z.B. bei 40 bis 300 bar oder bei 50 bis 200 bar.

Es ist möglich, die Umsetzung des cyclischen Olefins mit Distickstoffmonoxid bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden. Insbesondere ist Druck während der Umsetzung aber im Wesentlichen konstant.

Hinsichtlich der für die Umsetzung (im Labor - oder Produktionsmaßstab) einsetzbaren Reaktoren gibt es keine besonderen Beschränkungen. Insbesondere kann die Umsetzung in Batch-Fahrweise oder in kontinuierlicher Fahrweise erfolgen. Folglich können beispielsweise als Reaktoren mindestens ein CSTR (Continuous Stirred Tank Reactor) mit mindestens einem internen und/oder mindestens einem externen Wärmetauscher, mindestens ein Rohrreaktor, mindestens ein Rohrbündelreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden. Insbesondere wird die Umsetzung mit Distickstoffmonoxid in einem einzigen Reaktor durchgeführt. Beispielsweise kann die Umsetzung in kontinuierlicher Fahrweise oder in Batch-Fahrweise erfolgen.

Die Verweilzeit der Reaktionsmischung im Reaktor liegt im allgemeinen im Bereich von 0,1 bis 40 Stunden, bevorzugt im Bereich von 1 bis 30 Stunden, weiter bevorzugt im Bereich von 2 bis 25 Stunden.

Im Feed liegt das Molverhältnis von Distickstoffmonoxid und dem cyclischen Olefin im Allgemeinen im Bereich von 0,01 bis 30, wie z.B. im Bereich von 0,03 bis10, besonders bevorzugt im Bereich von 0,05 bis 1 und ganz besonders bevorzugt im Bereich von 0,08 bis0,2.

Das gewünschte Reaktionsprodukt entsteht dabei als Nebenkomponente, so dass das Reaktionsgemisch in geeigneter Weise aufzureinigen ist. Dies kann z.B. destillativ (wie insbesondere durch fraktionierte Destillation, vorzugsweise bei vermindertem Druck) oder chromatographisch erfolgen. Geeignete Reinigungsmethoden sind dem Fachmann geläufig. Die Aufreinigung kann z.B. batchwiese als auch kontinuierlich erfolgen.

Beispielsweise kann die Destillation mittels Destillationskolonne mit dem Fachmann bekannten Packungen verwendet. Die optimalen Destillationsbedingungen sind vom Fachmann ohne unzumutbaren Aufwand festlegbar. Die Destillation kann insbesondere im Vakuum, beispielsweise bei einem Druck < 1000 mbar, < 500 mbar, < 300 mbar, < 100 mbar oder < 10 mbar durchgeführt werden. Die verwendete Destillationskolonne kann mehrere, wie z.B. mindestens 20, mindestens 25 oder mindestens 30 theoretische, wie z.B. bis zu 70 Trennstufen aufweisen. Das Rücklaufverhältnis kann z.B. im Bereich von etwa 5 bis 100 liegen und mindestens 20, mindestens 25 oder mindestens 30 betragen und beträgt insbesondere etwa 100 für eine besonders vorteilhafte Fraktionierung.

Beispielsweise kann auch eine Säulenchromatographie an Stelle oder im Anschluss an eine destillative Reinigung erfolgen. Hierzu verwendet man dem Fachmann bekannte Säulenmaterialen und Laufmittel. Die optimalen Chromatographiebedingungen, wie Säulengeometrie und Geschwindigkeit des Laufmittels, sind vom Fachmann ohne unzumutbaren Aufwand festlegbar.

Beispiele für geeignete Säulenmaterialien sind polare Adsorptionsmittel wie z. B. Eisenoxid Fe₂O₃, Aluminiumoxid, Kohlenhydrate oder Kieselgel mit oder ohne Zusätze wie z. B. Fluoreszenzindikatoren oder Gips.

Beispiele für geeignete Laufmittel sind: aliphatische oder aromatische Laufmittel, wie z.B. Alkane oder Cycloalkane, wie z.B. Pentan, Petrolether, Hexan, Heptan, Toluol oder die korrespondierenden cyclischen Verbindungen; aliphatische Ether, Ester oder, wie z.B. Et₂O, MTBE EtOAc, Acetonoder Mischungen solcher Laufmitteln, wie z. B. Hexan/MTBE, Hexan/EtOAc, Pentan/Et₂O, Petrolether/Et₂O.

Man kann dabei einen gewünschten Carbaldehyd der Formel X, oder Gemische davon, in Reinform oder in einer Reinheit von mehr als 20, wie z.B. mehr als 30 mehr als 40, mehr als 50, mehr als 60, mehr als 60 oder mehr als 80 Gew.-%. isolieren

Der Carbaldehyd der Formel X kann dabei in stereoisomerenreiner Form, oder insbesondere als Gemisch zweier oder mehrerer Stereoisomeren, insbesondere wenn Rest A eine C=C Doppelbindung aufweist, isoliert werden

Insbesondere sind auf diesem Weg Aromastoffe enthaltend trans-Cyclopentadec-8-enylcarbaldehyd (I) und/ oder cis-Cyclopentadec-7-enylcarbaldehyd (III) zugänglich.

### Syntheseweg 2: Mehrstufige Carbaldehyd-Synthese über Wolff-Umlagerung

Ausgehend vom korrespondierenden von cycloaliphatischen Aldehyd, insbesondere einen cyclischen einfach oder mehrfach ungesättigen Aldehyd XII (d.h. im Vergleich zum cyclischen Rest A im Produkt der Formel X enthält die Ausgangsverbindung XII ein zusätzliches Ring-Kohlenstoffatom) sind Verbindungen der Formel X in einem mehrstufigen Verfahren zugänglich. Die einzelnen Synthesestufen sind dem Fachmann auf dem Gebiet der organischen Synthese an sich bekannt.

Beispiele für geeignete Ausgangsverbindungen (die sowohl in stereoisomerenreiner Form als auch in Form von Stereoisomeren-Gemischen eingesetzt werden können) zur Herstellung von Verbindungen der Formel (X), worin A für einen einfach ungesättigten C₁₅-Rest steht, umfassen Globanon , wie z.B. erhältlich von der Firma Symrise

### a) Stufe 1: Formylketon-Herstellung

Entsprechende Umsetzungen sind z.B. beschrieben in: Wu, Z.; Li, Y.; Cai, Y.; Yuan, J.; Yuan, C., Bioorganic & Medicinal Chemistry Letters 2013, 23, 4903-4906; oder Prelog, V.; Ruzieka, L.; Metzler, O, HELV. CHIM. ACTA 1947, 30, 1883-1895.

In einem geeigneten Reaktionsgemäß mit Rückflusskühler wird unter Inertgasatmosphäre eine Suspension eines geeigneten Hydrids, wie z.B. NaH, in organischer Phase, wie z.B. DMF, vorgelegt. Zu dieser Suspension wird bei verminderter Temperatur, wie z.B. 0 bis 10 °C eine Lösung des cycloaliphatischen Aldehyds, wie z.B. des einfach ungesättigten cyclischen C16-Ketons Globanon, z.B. über einen Zeitraum von 1 bis 5 Stunden zugetropft. Man setzt den Aldehyd in etwa in äquimolaren Menge, insbesondere aber in geringem (z.B. 1,1- bis 1,5 - fachem) molaren Unterschuss bezogen auf das Hydrid ein. Zur Vervollständigung der Deprotonierung kann die Temperatur erhöht und der Reaktionsansatz über eine geeignete Zeitspanne bei dieser Temperatur gehalten werden, z.B. für die Dauer von 1 bis 4 h bei 40 bis 80°C, wie z.B. 2h bei 60 °C. Dann wird der Reaktionsansatz wieder gekühlt, z.B. auf 0 bis 10 °C. Anschließend erfolgt die Zugabe einer Lösung aus Methylformiat in einem geeigneten Lösungsmittel, wie z.B. DMF. Man setzt Methylformiat in etwa in äquimolaren Menge, insbesondere aber in geringem (z.B. 1,1- bis 1,5 -fachem) molaren Überschuss bezogen auf den Aldehyd ein. Das Reaktionsgemisch wird über einen geeigneten Zeitraum, wie z.B. 5 bis 30 h, wie z.B. etwa 16 h bei Umgebungstemperatur gerührt und die Reaktion anschließend durch Zugabe von Eiswasser beendet. Die Mischung wird mit Ether, z.B. MTBE, gewaschen und die organischen Phasen werden verworfen. Durch Zugabe von Schwefelsäure wird die wässrige Phase angesäuert, z.B. auf pH 2, und mehrmals mit Essigester extrahiert. Die vereinigten Essigesterphasen werden über Na₂SO₄ getrocknet und anschließend im Vakuum eingeengt. Der so erhaltene Rückstand besteht zum überwiegenden Teil aus den gewünschten regioisomeren Formylketonen XII bzw. deren tautomeren Enolen und wird ohne weitere Aufarbeitung in der nachfolgenden Stufe eingesetzt.

### b) Stufe 2: Diazoketon-Herstellung

Entsprechende Umsetzungen sind z.B. beschrieben in Regitz, M.; und Ruter, J., Chem. Ber. 1968, 101, 1263 - 1270.

Zu einer in einem geeigneten Reaktionsgefäß vorgelegten Lösung des Rohproduktes aus der vorhergehenden Stufe 1 und Triethylamin, (insbesondere in molarem Überschuss, wie z.B. 1,5-bis 2-facher Überschuss) in einem geeigneten Lösungsmittel, insbesondere in einem halogenierten Kohlenwasserstoff, wie Dichlormethan, wird unter Kühlung, z.B. auf -20 bis 0°C, insbesondere bei -10 °C eine Lösung von 4-Acetamidobenzoesäureazid (in molarem Überschuss, wie z.B. 1,5- bis 2-facher Überschuss) im gleichen Lösungsmittel über einen Zeitraum von mehreren Stunden, wie z.B. 2 bis 8 h zugetropft. Grundsätzlich sind auch andere Diazotransferreagenzien geeignet, wie Toulolsulfonsäureazid. Gegebenenfalls nach Stehenlassen des Reaktionsansatzes bei erhöhter Temperatur, wie z.B. Raumtemperatur und Lösungsmittelaustausch (z.B. Dichlormethan durch MTBE) wird die organische Phase mit Natronlauge gewaschen und die wässrige Phase zweimal mit dem organischen Lösungsmittel, z:B. MTBE extrahiert. Die vereinigten organischen Phasen, enthaltend das gewünschte Diazoketon XIV (ggf. als Isomerengemisch), werden über Na₂SO₄ getrocknet und wie nachfolgend beschrieben weiter umgesetzt.

### c) Stufe 3: Wolff-Umlagerung und Veresterung des dabei gebildeten Ketens:

Entsprechende Umsetzungen sind z.B. beschrieben in Regitz, M.; und Ruter, J., Chem. Ber. 1968, 101, 1263 - 1270 oder Kirmse, W., Eur. J. Org. Chem. 2002, 14, 2193_2256.

Eine Lösung des Diazoketons aus Stufe 2 wird langsam über einen Zeitraum von 5 bis 20 h, wie z.B. 10 h auf erhitzten Alkanol, wie z.B. erhitztes 1-Hexanol (z.B. 150 °C), vorgelegt in einem mit Destillationsbrücke versehenen Reaktionsgefäß getropft. Unter kontrollierter N₂-Bildung wird gleichzeitig das organische Lösungsmittel abdestilliert. Am Ende der Gasentwicklung wird die Reaktionsmischung abgekühlt und anschließend werden Reste von von Lösungsmittel und Alkanol im Vakuum entfernt und man erhält den gewünschten cyclischen Ester XV, mit einem um 1 Kohlenstoffatom verkleinerten aliphatischen Ring (ggf in Form eines Isomerengemischs) enthält.

### d) Stufe 4: Esterreduktion zum Alkohol

Entsprechende Umsetzungen sind z.B. beschrieben in March, J. "Advanced organic Chemistry", 4th edition, John Wiley & Sons, New York 1992.

Zu einer Lösung des Esters aus Stufe 3 wird in einem inerten organischen apolaren Lösungsmittel, wie z.B. Toluol, vorgelegt in einen gekühlten Reaktionsgefäß, z.B. bei -78 °C, ein Reduktionsmittel, z.B. eine Lösung von Diisobutylaluminiumhydrid (DIBAL-H) in molarem Überschuss (z.B. 1,5- bis 3-fach) getropft. Die Kühlung wird entfernt und nach Stehenlassen bei Umgebungstemperatur wird die Reaktion beendet, wie z.B. durch Zugabe von Ethylacetat und einer gesättigten wässrigen K,Na-Tartratlösung. Die wässrige Phase wird mit Ethylacetat extrahiert, die vereinigten organischen Phasen über Na₂SO₄ getrocknet und im Vakuum eingeengt, wobei man die korrespondierenden cyclischen Alkohole XVI (ggf in Form eines Isomerengemischs) erhält

### e) Stufe 5: Alkoholoxidation zum Carbaldehyd:

Entsprechende Umsetzungen sind z.B. beschrieben in March, J. "Advanced organic Chemistry", 4th edition, John Wiley & Sons, New York 1992.

Zu einer Lösung des Reaktionsprodukts aus Stufe 4 in einem geeigneten Lösungsmittel, wie z.B. Dichlormethan, werden nacheinander Kieselgur und ein für primäre Alkohole geeignetes Oxidationsmittel, wie z.B. HOCI, Pyridiniumdichromat, oder insbesondere Pyridiniumchlorochromat (PCC) (in etwa in äquimolaren Mengen) gegeben. Nach mehrstündiger Reaktion, z.B. 3-6 h bei Umgebungstemperatur, wird die Reaktionsmischung über Kieselgel filtriert und im Vakuum eingeengt. Man erhält den gewünschten Aldehyd X (ggf in Form eines Isomerengemischs).

### e) Stufe 6 (optional) Säulenchromatographie

Hierzu verwendet man dem Fachmann bekannte Säulenmaterialen und Laufmittel. Die optimalen Chromatographiebedingungen, wie Säulengeometrie und Geschwindigkeit des Laufmittels, sind vom Fachmann ohne unzumutbaren Aufwand festlegbar.

Beispiele für geeignete Säulenmaterialein sind polare Adsorptionsmittel wie z. B. Eisenoxid Fe₂O₃, Aluminiumoxid, Kohlenhydrate oder Kieselgel mit oder ohne Zusätze wie z. B. Fluoreszenzindikatoren oder Gips.

Beispiel für geeignete Laufmittel sind: aliphatische oder aromatische Laufmittel, wie z.B. Alkane oder Cycloalkane, wie z.B. Pentan, Petrolether, Hexan, Heptan, Toluol oder die korrespondierenden cyclischen Verbindungen; aliphatische Ether, Ester oder Ketone, wie z.B. MTBE, Et₂O, EtOAc oder Aceton oder Mischungen solcher Laufmittel wie z. B. Hexan/MTBE, Hexan/EtOAc, Pentan/Et₂O, Petrolether/Et₂O.

Man kann dabei einen gewünschten Carbaldehyd, oder Gemische davon, in Reinform oder in einer Reinheit von mehr als 20, wie z.B. mehr als 30 mehr als 40, mehr als 50 , mehr als 60, mehr als 60 oder mehr als 80 Gew.-%. isolieren

Der Carbaldehyd kann dabei in stereoisomerenreiner Form, oder insbesondere als Gemisch zweier oder mehrerer Stereoisomeren vorliegen.

Insbesondere sind auf diesem Weg Aromastoffe, enthaltend trans-Cyclopentadec-8-enylcarbaldehyd (I) und/ oder cis-Cyclopentadec-7-enylcarbaldehyd (III), und/oder trans-Cyclopentadec-7-enylcarbaldehyd (II); insbesondere ternäre Mischungen davon zugänglich.

Die Erfindung wird nun unter Bezugnahme auf folgende nichtlimitierende Ausführungsbeispiele näher erläutert:

### Experimenteller Teil

### Methoden:

### Gaschromatographie (GC)

Trennsäule : CP-Wax 52CB 25m x 0,32mm x1,2µm 1ml/min N₂
Bedingungen: 90°-5min-10°/min-240°-30min Inj/Det 200°/250° (Methode A)
Bedingungen: 80°-3°/min-250°- Inj/Det 200°/250° (Methode B) (Nur Beispiel 2)
Probenvolumen: 0,2 ml

### GC/MS

Trennsäule: CP-Wax 52 CB (1.2 µm Filmdicke), Splitverhältnis 10:1
Bedingungen: 80°-3min-240°-30min 0.2µl
MS-Bedingungen: 25-785 amu, 70 eV

### G C/I R

Detektor: MCT/AWellenlänge 650 - 4000 cm⁻¹
Zellen-/Transfertemperatur 250 °C
Scan 6
Resolution 8

### Säulenchromatographie

Verwendet wurde eine Glassäule mit Frittenboden. Die Säule wurde zu 2/3 gepackt mit aufgeschlemmten Kieselgel F₂₅₄. Das Lösungsmittelgemisch wurde mit einem Überdruck von 0.2-0.4 bar durch die Säule gedrückt.

### Beispiel 1: C15-Aldehyd-Synthese durch N₂O-Oxidation von 1,8-Cyclohexadecadien

In einem adiabaten Rohrreaktor (3 m Länge, Durchmesser 6 cm, Reaktorvolumen 9 L) gefüllt mit Raschigringen aus 1.4541 Edelstahl wurden bei 216 °C Reaktoreingangstemperatur 2000 g/h 1,8-Cyclohexadecadien (cis/trans-Isomerengemisch) mit 52 mL/h einem N₂O/CO₂ Gemisch (15% CO₂ Anteil) umgesetzt. Molverhältnis N₂O/Olefin 9-10 Nicht umgesetztes 1,8-Cyclohexadecadien wurde mittels einer Destillationskolonne (Montz-Gewebepackung A3, trennwirksame Höhe 4000 mm, Innendurchmesser 55 mm, Zulauf auf halber Höhe der Kolonne) bei 210 °C Sumpftemperatur und einem Kopfdruck von 20 mbar destillativ abgetrennt. Der Sumpfaustrag enthält ca. 5 Gew.-% der C15-Aldehyde (I) (t_{Ret} =23,3 min GC Methode A) und (III) (t_{Ret} =24.1 min) und weniger als 1 Gew.-% von 1,8-Cyclohexadecadien (t_{Ret} = 18,274" 18,516, 18,946; 3 Isomere) (Hauptkomponente im Sumpf ist Globanon; t_{Ret} =24,433, 24,708, 2 Isomere).

### Beispiel 2: C15-Aldehyd-Synthese durch N₂O-Oxidation von 1,9-Cyclohexadecadien

Eine Mischung bestehend aus Cyclohexadeca-1,9-dien (Isomerengemisch, Summe der Isomere ca. 98%, 2 g) und Cyclohexan (98.5 g) wurden in einem 300 ml Autoklav gemischt. Der Autoklav wurde verschlossen und mit Stickstoff auf 50 bar aufgepresst und anschließend wieder entspannt. Der Vorgang wurde dreimal wiederholt. Nach der letzten Entspannung wurde der Autoklav mit N₂O auf 30 bar aufgepresst, Molverhältnis N₂O/Olefin 25-27 danach der Rührer eingeschaltet (mit 400 U/min) und der Autoklav innerhalb 1 Stunde auf die Reaktionstemperatur aufgeheizt. Die Mischung wurde für 24 h bei 240 °C gerührt. Anschließend wurde auf Raumtemperatur abgekühlt, der Autoklav entspannt und der Rohaustrag per GC analysiert. In Summe enthielt der Austrag 3.5 Gew.-% trans-Cyclopentadec-8-enylcarbaldehyd (I) (t_{Ret} =23,3 min) und cis-Cyclopentadec-7-enylcarbaldehyd (III). (t_{Ret} =24,1 min)

### Beispiel 3: Anreicherung von C15-Aldehyden durch fraktionierte Destillation

2600 g eines Gemisches (Rohaustrag aus Oxidation von Cyclohexadeca-1,8-dien mit N₂O; s. Beispiel 1) mit in Summe ca. 5% trans-Cyclopentadec-8-enylcarbaldehyd (I) und *cis-*Cyclopentadec-7-enylcarbaldehyd (III) wurden in einer Batchkolonne (Sulzer-Gewebepackung DX, trennwirksame Höhe 2000 mm, Durchmesser 43 mm, Kopfdruck: 5 mbar, Druckverlust über Kolonne: 5 mbar, Sumpftemperatur: 180°C, Sambayverdampfer, Rücklaufverhältnis: 100) fraktioniert destilliert. Das Gemisch aus *trans*-Cyclopentadec-8-enylcarbaldehyd (I) (t_{Ret} =23,3 min) und *cis*-Cyclopentadec-7-enylcarbaldehyd (III) (t_{Ret} =24,1 min) konnte dabei in verschiedenen Fraktionen auf 29, 33 und 46 Gew.-% angereichert werden. Der jeweilige Gehalt wurde gaschromatographisch bestimmt. (Siedepunkte der Fraktionen 160, 167 und 167 °C.)

### Beispiel 4: Aufreinigung durch Säulenchromatographie

2.05 g eines Gemisches aus *trans*-Cyclopentadec-8-enylcarbaldehyd (I) und *cis-*Cyclopentadec-7-enylcarbaldehyd (III) mit einem Gehalt in Summe von 33 Gew.-% (vgl. Beispiel 3) wurden mittels Säulenchromatographie (Kieselgel, Glassäule mit Frittenboden, Überdruck von 0.2 bar.) unter Verwendung eines Laufmittelgemisches Cyclohexan/MTBE (50:1 und 40:1) gereinigt. Nach chromatographischer Reinigung wurden 100 mg *trans-*Cyclopentadec-8-enylcarbaldehyd (I) (t_{Ret} =23,494 min) mit einer Reinheit von 83% isoliert. 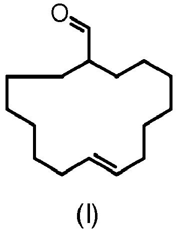 ¹H NMR (500 MHz, CDCl₃, 25 °C): σ = 9.5 (s, CHO, 1H), 5.4-5.3 (m, HC=CH, 2H), 2.4-2.3 (m, 1H), 2.2-1.9 (m, 4H), 1.7-1.6 (m, 2H), 1.5-1.1 (m, 18H).
¹³C-NMR (125 MHz, CDCl₃, 25 °C): σ = 206.0 (C=O), 131.2 (HC=CH), 48.4 (CH), 31.6 (2xCH₂), 28.3 (2xCH₂), 27.0 (2xCH₂), 26.7 (2xCH₂), 26.6 (2xCH₂), 25.1 (2xCH₂).
IR (GC/IR) u [cm⁻¹] = 3029 (1,2 trans subst. DB), 2934, 2865, 2797, 2695, 1738 (CHO), 1454, 1353, 1110, 968 (1,2 trans subst. DB).

### Beispiel 5: C15-Aldehyd-Synthese über Wolff-Umlagerung

### a) Stufe 1:

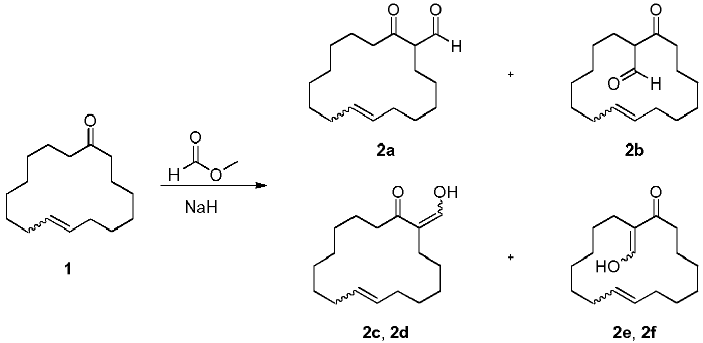

In einem Dreihalskolben mit Rückflusskühler und mechanischem Rührer wird unter Argonatmosphäre NaH (60 Gew.-% in Mineralöl, 33.5 g, 0.84 mol, 1.1 Äq.) vorgelegt und in DMF (DMF= *N,N*-Dimethylformamid, 50 mL) suspendiert. Zu dieser Suspension wird bei 0 °C eine Lösung aus Globanon (1, 150 g, 0.64 mol, 1.3 Äq.) in trockenem DMF (250 mL) über einen Zeitraum von 3.5 h zugetropft. Zur Vervollständigung der Deprotonierung wird für 2 h auf 60 °C erhitzt und anschließend auf 0 °C gekühlt. Nach Zugabe einer Lösung aus Methylformiat (57 g, 0.95 mol,1.5 Äq.) in DMF (50 mL) wird für 16 h bei Umgebungstemperatur gerührt und die Reaktion anschließend durch Zugabe von Eiswasser beendet. Die Mischung wird zweimal mit MTBE (MTBE: Methy-*tert*-butylether) gewaschen und die organischen Phasen verworfen. Durch Zugabe von Schwefelsäure (50 Gew.-%ig) wird die wässrige Phase auf pH 2 gebracht und mehrmals mit Essigester extrahiert. Die vereinigten Essigesterphasen werden über Na₂SO₄ getrocknet und anschließend im Vakuum eingeengt. Der so erhaltene Rückstand (134 g) besteht It. NMR zu etwa 90% aus den gewünschten regioisomeren Formylketonen 2a und 2b bzw. deren tautomeren Enolen (2c-2f) und wird ohne weitere Aufarbeitung in der nachfolgenden Stufe eingesetzt.

### b) Stufen 2 und 3:

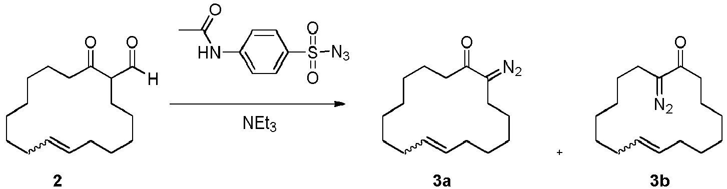

Zu einer Lösung des Rohproduktes (2a-f) aus der vorhergehenden Stufe 1 (134 g Reinheit etwa 90%, 0.46 mol, 1 Äq.) und Triethylamin (86.6 g, 0.86 mol, 1.9 Äq.) in Dichlormethan (100 mL) wird bei -10 °C eine Lösung von 4-Acetamidobenzoesäureazid (97.3 g, 0.41 mol, 0.9 Äq.) in Dichlormethan (400 mL) über einen Zeitraum von 4 h zugetropft. Nach 20 h bei RT wird MTBE (400 mL) zugegeben und das Dichlormethan abdestilliert. Die organische Phase wird mit Natronlauge (6 Gew.-%) gewaschen und die wässrige Phase zweimal mit MTBE extrahiert. Die vereinigten MTBE-Phasen werden über Na₂SO₄ getrocknet und wie nachfolgend beschrieben weiter umgesetzt.

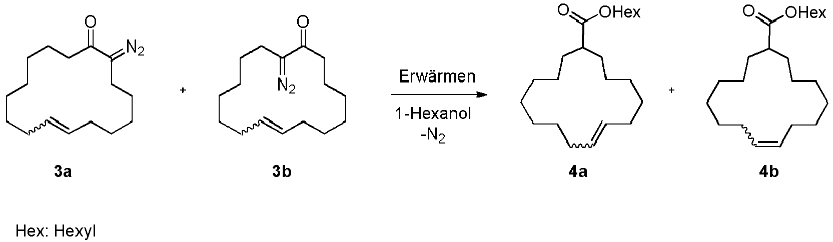

Die Lösung des Diazoketons 3 in MTBE wird langsam über einen Zeitraum von 10 h auf 150 °C erhitztes 1-Hexanol (300 mL) getropft, dass die N₂-Bildung kontrollierbar bleibt und gleichzeitig der eingetragene MTBE kontinuierlich über eine Destillationsbrücke abdestilliert werden kann. Am Ende der Gasentwicklung wird die Reaktionsmischung abgekühlt und anschließend Reste von MTBE und das 1-Hexanol im Vakuum entfernt. Es werden 92 g eines gelben, zähflüssigen Rückstandes erhalten, der It. NMR 60-70 % der Ester 4a und 4b als Isomerengemisch enthält.

### c) Stufe 4:

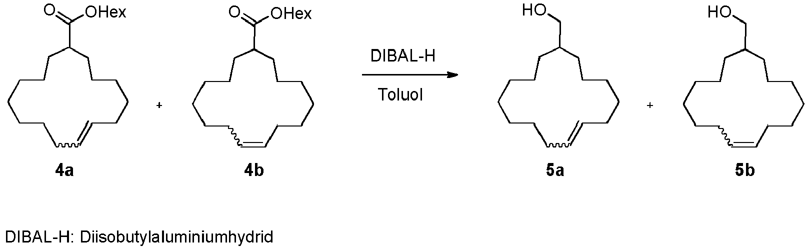

Zu einer Lösung des Esters (4a und 4b) (92 g, 70% Reinheit, 0.2 mol, 1Äq.) in Toluol (130 mL) wird bei -78 °C eine Lösung von Diisobutylaluminiumhydrid (DIBAL-H) in Heptan (c = 1 mol/L, 490 mL, 0.5 mol, 2.5 Äq.) getropft. Die Kühlung wird entfernt und nach 2 h bei Umgebungstemperatur wird die Reaktion durch Zugabe von Ethylacetat (50 mL) und gesättigter wässriger K,Na-Tartratlösung (400 mL) beendet. Die Suspension wird bis zur Aufhebung der Trübung bei RT gerührt. Die wässerige Phase wird mit Ethylacetat extrahiert, die vereinten organischen Phasen über Na₂SO₄ getrocknet und im Vakuum eingeengt. Es werden 77 g der isomeren Alkohole **5a** und **5b** als farbloses Öl erhalten.

### d) Stufe 5:

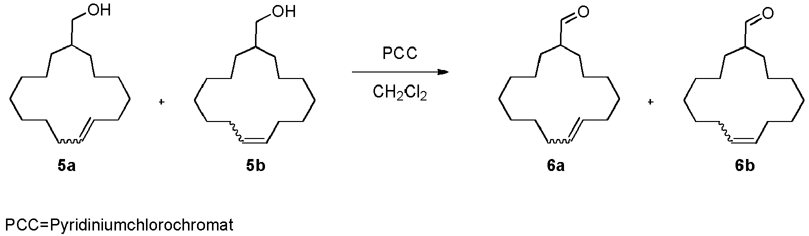

Zu einer Lösung der Hälfte des Rohgemisches der vorangehenden Stufe 4 (33 g, 0.14 mol, 1 Äq.) in Dichlormethan (300 mL) werden nacheinander Kieselgur (30 g) und Pyridiniumchlorochromat (PCC) (30 g, 0.14 mol, 1 Äq.) gegeben. Nach 4 h bei Umgebungstemperatur wird die Reaktionsmischung über Kieselgel filtriert und im Vakuum eingeengt. Es werden 21 g der isomeren Aldehyde **6a** und **6b** in einer Reinheit von 80% erhalten (71 mmol, Gesamtausbeute über fünf Stufen ausgehend von Globanon 22%).

Zur Bewertung der olfaktorischen Eigenschaften wird ein Teil des Rohproduktes durch Säulenchromatographie gereinigt.

### e) Stufe 6 (Säule Isolierung I, II und III)

5.0 g eines Gemisches aus trans-Cyclopentadec-8-enylcarbaldehyd (I), trans- und cis-Cyclopentadec-7-enylcarbaldehyd (II) und (III) (Verhältnis von (I) : (II) : (III) = 43 : 23 : 33) mit einem Gehalt in Summe von 70 Gew.-% wurden mittels Chromatographie unter Verwendung eines Laufmittelgemisches Cyclohexan/MTBE (60:1 - 40:1) gereinigt. Nach chromatographischer Reinigung wurden 1 g eines Gemisches aus trans-Cyclopentadec-8-enylcarbaldehyd (I), trans- und cis-Cyclopentadec-7-enylcarbaldehyd (II) und (III) mit einer Reinheit von 87% (Summe von (I)+(II)+(III) im Verhältnis 46 : 24 : 29) isoliert.

### f) Analysenergebnisse:

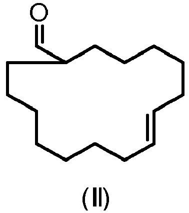

IR (GC/IR) u [cm⁻¹] = 3023 (trans 1,2 subst. DB), 2934, 2864, 2801, 2696, 1738 (CHO), 1454, 968 (trans 1,2 subst. DB).
MS (GC/MS-IR-Kopplung) m/z = 236. 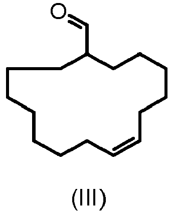 IR (GC/IR) u [cm⁻¹] = 3012 (1,2 cis subst., DB), 2935, 2866, 2801, 2698, 1738 (CHO), 1457, 719 (1,2 cis subst. DB).
MS (GC/MS-IR-Kopplung) m/z = 236.

### NMR-Daten des Gemisches (II) und (III):

¹H NMR (500 MHz, CDCl₃, 25 °C): σ = 9,6 (s, 2H, 2 x CHO), 5.4-5.2 (m, 4H, 2 x HC=CH), 2.5-1 (m, 50H).
¹³C-NMR (125 MHz, CDCl₃, 25 °C): σ =205.4 (CHO), 205.3 (CHO), 131.4, 131.0, 130.2 (2xCHₐᵣ), 50.4 (CHₐₗₖ), 49.9 (CHₐₗₖ), 31.7 - 24.4 (24 x CH₂).

Figur 1 zeigt die GC/IR Spektren der erfindungsgemäßen Verbindungen I, II und II (dort bezeichnet als Komponenten 1, 2 und 3)

### Beispiel 6: Olfaktorische Bewertung

### 1) Bewertung verschiedener Gemische der Aldehyde I, II und III

### Zusammensetzung der untersuchten Proben:

Nr 1: (I) : (II) : (III) = 46 : 24 : 29; Reinheit = 87.5% (Summe (I-III)) (Vgl. Beispiel 5, nach Stufe 6)
Nr.2: (I) : (II) : (III) = 43 : 24 : 33; Reinheit = 77.8% (Summe (I-III))

### Ergebnis

### Probe Nr. 1:

| | |
|---|---|
| Riechstreifentest <1min | Moschus |
| Riechstreifentest 30min | Moschus |
| Riechstreifentest 1h | Moschus |
| Riechstreifentest 24h | Moschus |

### Probe Nr. 2:

| | |
|---|---|
| Riechstreifentest <1min | Moschus (deutlich intensiver als Probe Nr. 1) |
| Riechstreifentest 30min | Moschus (deutlich intensiver als Probe Nr. 1) |
| Riechstreifentest 1h | Moschus (deutlich intensiver als Probe Nr. 1) |
| Riechstreifentest 24h | Moschus (deutlich intensiver als Probe Nr. 1) |

Die Gemische der Aldehyde (I-III) haben eine moschusartige Geruchsnote.

### 2) Bewertung des Aldehyds I:

### Zusammensetzung der Probe

(I) : (II) : (III) = 93 : 2 : 4; Reinheit = 89.4% (Summe (I-III)) (vgl. Beispiel 4)

### Ergebnis:

| | |
|---|---|
| Riechstreifentest <1min | Moschus , deutlich seifig, grün, gasig |
| Riechstreifentest 10min | Moschus deutlich seifig, grün, gasig |
| Riechstreifentest 30min | Moschus deutlich seifig, grün, gasig |
| Riechstreifentest 1h | Moschus deutlich seifig, grün, gasig |
| Riechstreifentest 24h | Moschus |

Die Reinsubstanz **(I)** hat neben der moschusartigen Geruchsnote auch einen deutlich seifigen, grünen und gasigen Geruch.

Auf die Offenbarung der hierin erwähnten Druckschriften , wird ausdrücklich Bezug genommen.

## Patentansprüche

1. Carbaldehyd der allgemeinen Formel X 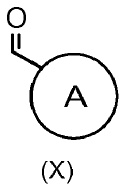 worin A für einen cycloaliphatischen Kohlenwasserstoffrest mit m Ringkohlenstoffatomen, wobei m für einen ganzzahligen Wert von 13 bis 17 steht, und gegebenenfalls n C=C-Doppelbindungen aufweist, wobei n für einen ganzzahligen Wert von 1, 2 oder 3 steht, steht.

2. Verbindung nach Anspruch 1, worin n für 1 steht und/oder m für 15 steht.

3. Verbindung nach Anspruch 2, ausgewählt unter den isomeren Verbindungen der Formeln I, II und III 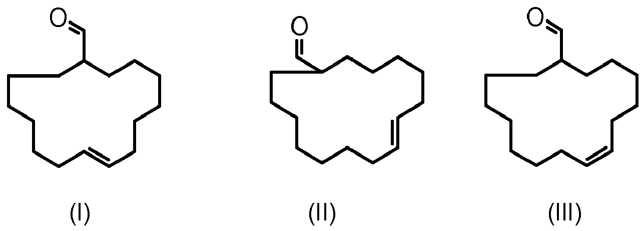 und den stereoisomeren Formen davon

4. Stoffgemisch umfassend wenigsten eine Verbindung nach einem der Ansprüche 1 bis 3

5. Verwendung wenigstens eines Stoffes oder Stoffgemisches nach einem der vorhergehenden Ansprüche als Aromachemikalie, insbesondere als Riechstoff.

6. Verwendung nach Anspruch 5 in Mitteln, ausgewählt unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.

7. Verwendung nach einem der Ansprüche 5 und 6 eines Gemischs, im Wesentlichen enthaltend trans-Cyclopentadec-8-enylcarbaldehyd (I).

8. Verwendung nach Anspruch 7, eines Gemisches enthaltend trans-Cyclopentadec-8-enylcarbaldehyd (I), trans-Cyclopentadec-7-enylcarbaldehyd (II) und *cis*-Cyclopentadec-7-enylcarbaldehyd (III), wobei im Gemisch der Anteil von trans-Cyclopentadec-8-enylcarbaldehyd (I) bezogen der Summe der Komponenten der Formeln I, II und III mindestens 65% wie z.B. 65-100%, 75-98% oder 85-95% beträgt.

9. Verwendung nach Anspruch 5 oder 6 eines Gemisches enthaltend die Verbindungen der Formeln I, II und III, wobei das Gewichtsverhältnis von I : II : III im Bereich von 0,3-0,5 : 0,2-0,3 : 0,3-0,4 liegt.

10. Verwendung nach Anspruch 9 eines Gemisches enthaltend die Verbindungen der Formeln I, II und III in einem Gewichtsverhältnis von I : II : III, von etwa 43 : 24 : 33.

11. Verwendung nach einem der Ansprüche 5 bis 10 zur Erzeugung einer Moschusnote in einer Riechstoffzusammensetzung.

12. Verwendung nach Anspruch 11, zum Vermitteln, Modifizieren und/oder Verstärken einer Moschusduftnote in einer Riechstoffzusammensetzung durch Beimischen einer sensorisch wirksamen Menge wenigstens eines Stoffs oder ein Stoffgemischs gemäß der Definition in einem der Ansprüche 1 bis 10.

13. Verfahren zur Herstellung von einer Verbindung der allgemeinen Formel X 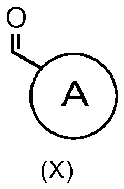 worin A für einen cycloaliphatischen Kohlenwasserstoffrest mit m Ringkohlenstoffatomen, wobei m für einen ganzzahligen Wert von 13 bis 17 steht, und gegebenenfalls n C=C-Doppelbindungen aufweist, wobei n für einen ganzzahligen Wert von 1, 2 oder 3 steht, wobei man
a) eine cycloaliphatische Verbindung der Formel XI 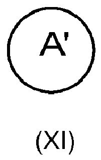 worin A' für einen cycloaliphatischen Kohlenwasserstoffrest mit m+1 Ringkohlenstoffatomen, wobei m für einen ganzzahligen Wert von 13 bis 17 steht, und gegebenenfalls n+1 C=C-Doppelbindungen aufweist, wobei n für einen ganzzahligen Wert von 1, 2 oder 3 steht, mit Distickstoffmonoxid oxidiert; und
b) wenigstens eine Verbindung der Formel X aus dem Reaktionsgemisch isoliert.

14. Verfahren nach Anspruch 13, wobei man die Verbindung der Formel X durch Destillation und gegebenenfalls anschließende Säulenchromatographie isoliert.

15. Verfahren nach Anspruch 13 oder 14, wobei man als Edukt der Formel XI ein 1,8- oder 1, 9-Cyclohexadecadien einsetzt und trans-Cyclopentadec-8-enylcarbaldehyd (I) und/oder *cis*-Cyclopentadec-7-enylcarbaldehyd (III), isoliert.

16. Verfahren nach einem der Ansprüche 13 bis 15, wobei man die Verbindung der Formel (I) und der Formel (III) mittels fraktionierter Destillation aus einem Reaktionsgemisch isoliert, worin das Gewichtsverhältnis von (I) plus (III) zu Cyclohexadeca-1,8-dien mindestens 0.01, wie z.B. 0.01-0.1, 0.03-0.095 oder 0.06-0.09 beträgt.

17. Verfahren nach einem der Ansprüche 13 bis 15, wobei man trans-Cyclopentadec-8-enylcarbaldehyd (I) mittels chromatischer Aufreinigung aus einem Gemisch isoliert, welches durch fraktionierte Destillation erhalten wurde, und einen Gehalt an (I) plus (III) von mindestens 5 Gew.-%, wie z.B 5-50%, 20-48 oder 30-45%, aufweist.

18. Verfahren zur Herstellung von einer Verbindung der allgemeinen Formel X 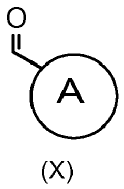 worin A für einen cycloaliphatischen, Kohlenwasserstoffrest mit m Ringkohlenstoffatomen, wobei m für einen ganzzahligen Wert von 13 bis 17 steht, und gegebenenfalls n C=C-Doppelbindungen aufweist, wobei n für einen ganzzahligen Wert von 1, 2 oder 3 steht, wobei man
a) eine cycloaliphatische Verbindung der Formel XII 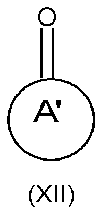 worin A' für einen cycloaliphatischen, Kohlenwasserstoffrest mit m+1 Ringkohlenstoffatomen, wobei m für einen ganzzahligen Wert von 13 bis 17 steht, und gegebenenfalls n+1 C=C-Doppelbindungen aufweist, wobei n für einen ganzzahligen Wert von 1, 2 oder 3 steht,
mit NaH und Methylformiat zum korrespondierenden cyclischen Formylketon XIII 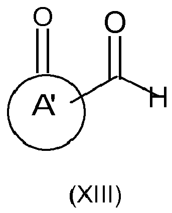 umsetzt; worin A' wie oben definiert ist;
b) das gebildete Formylketon mit Triethylamin und 4-Acetamidobenzoesäureazid zum korrespondierende Diazoketon XIV umsetzt; 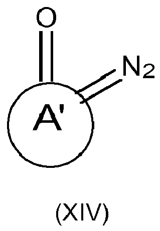 worin A' wie oben definiert ist;
c) aus dem gebildete Diazoketon unter den Bedingungen einer Wolff-Umlagerung N₂ abspaltet und in Gegenwart eines Alkanols zum korrespondierenden Ester der Formel XV 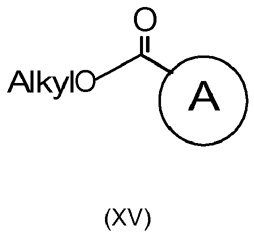 umsetzt; worin A wie oben definiert ist
d) den so gebildeten Ester zum korrespondierenden Alkohol XVI 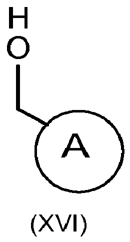 reduziert; worin A wie oben definiert ist;
e) den so gebildeten Alkohol zum Carbaldehyd der Formel X oxidiert;
f) und gegebenenfalls das Carbaldehyd der Formel X aus dem Reaktionsgemisch isoliert.

19. Verfahren nach Anspruch 18, wobei man (E/Z)-Cyclohexadec-8-enon als Verbindung der Formel XI in Stufe a) einsetzt und trans-Cyclopentadec-8-enylcarbaldehyd (I), *trans-*Cyclopentadec-7-enylcarbaldehyd (II) und *cis*-Cyclopentadec-7-enylcarbaldehyd (III), in Stufe a) erhält.

20. Verfahren nach Anspruch 15, wobei man Verbindungen der Formel (I), (II) oder (III) oder Gemische davon in Stufe f) durch chromatische Aufreinigung eines Reaktionsgemischs aus Stufe e) erhält, wobei dessen Gehalt an (I), (II) und (III) in Summe vorzugsweise mindestens 50 Gew.-% wie z.B. 50-100 Gew.-%, 60-99 Gew.-% oder 70-95 Gew.-% beträgt.

21. Riechstoffzusammensetzung, enthaltend wenigstens einen Stoff oder ein Stoffgemisch gemäß der Definition in einem der Ansprüche 1 bis 10 oder hergestellt nach einem der Verfahren gemäß den Ansprüchen 13 bis 20.

22. Zusammensetzung nach Anspruch 21, enthaltend den Stoff oder das Stoffgemisch in einem Gewichtsanteil von 0,01 bis 99,9 Gew.-% bezogen auf das Gesamtgewicht der Zusammensetzung.

23. Mittel enthaltend wenigstens einen Stoff oder ein Stoffgemisch gemäß der Definition in einem der Ansprüche 1 bis 10 oder hergestellt nach einem der Verfahren gemäß den Ansprüchen 13 bis 20.

24. Mittel nach Anspruch 23, enthaltend den Stoff oder das Stoffgemisch in einem Gewichtsanteil von 0,01 bis 99,9 Gew.-% bezogen auf das Gesamtgewicht der Zusammensetzung.

25. Mittel nach Anspruch 23 oder 24, ausgewählt unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.
